# EUROPÄISCHE PATENTSCHRIFT

(11) **EP 2 059 455 B1**
(45) Veröffentlichungstag und Bekanntmachung des Hinweises auf die Patenterteilung: **18.05.2011**
(21) Anmeldenummer: 07801773.8
(22) Anmeldetag: 06.09.2007
(51) Int. Cl.: B65D 75/34, B65D 75/58, A61J 1/03

(54) **VERPACKUNG FÜR WIRKSTOFFHALTIGE FILME UND VERFAHREN FÜR DEREN HERSTELLUNG**
PACKAGING FOR FILMS WHICH CONTAIN ACTIVE SUBSTANCES, AND METHOD FOR PRODUCING IT
EMBALLAGE POUR FILMS CONTENANT UNE SUBSTANCE ACTIVE, ET SON PROCÉDÉ DE FABRICATION

(30) Priorität: 07.09.2006 DE 102006041921
(43) Veröffentlichungstag der Anmeldung: 20.05.2009
(73) Patentinhaber: LTS LOHMANN Therapie-Systeme AG, 56626 Andernach (DE)
(72) Erfinder: KRUMME, Markus, Randolph, NJ 07869 (US)
(74) Vertreter: Flaccus, Rolf-Dieter
(86) Internationale Anmeldenummer: PCT/EP2007/007333
(87) Internationale Veröffentlichungsnummer: WO 2008/028560

(56) Entgegenhaltungen:
- EP-A- 1 602 593
- WO-A-99/54231
- DE-A1- 2 322 055
- DE-A1- 10 102 818
- DE-A1-102004 047 445
- DE-U1- 20 320 948
- US-A- 3 924 746
- US-A- 3 933 245

## Beschreibung

Die Erfindung betrifft Verpackungen für wirkstoffhaltige Filme, insbesondere für Filme mit pharmazeutischen Wirkstoffen. Sie betrifft ferner die Verwendung dieser Verpackungen zum Verpacken von wirkstoffhaltigen Filmen, sowie Herstellungsverfahren, mit denen die erfindungsgemäßen Verpackungen erhalten werden können. Die erfindungsgemäßen Verpackungen eignen sich insbesondere zum kindersicheren Verpacken von wirkstoffhaltigen Filmen.

In der Arzneimitteltherapie werden neben den bekannten Darreichungsformen, wie Tabletten, Kapseln etc., auch wirkstoffhaltige Filme, sogenannte "Wafer", zur Verabreichung von Arzneistoffen verwendet, vor allem zur oralen Verabreichung. Es handelt sich hierbei um dünne Plättchen oder Streifen aus einem wirkstoffhaltigen Film, wobei diese Plättchen oder Streifen in ihrer Dicke und den Abmessungen auf die abzugebende Wirkstoffmenge abgestimmt sind. Ein Wafer ist im allgemeinen flexibel, weich, von geringem Gewicht und reißfähig. Die Gesamtdicke eines derartigen filmförmigen Arzneimittels kann 5 µm bis 5 mm betragen, üblicherweise 50 µm bis 1 mm. Die Flächenform kann rund, oval, drei- oder viereckig, oder auch vieleckig gestaltet sein oder eine beliebig gerundete Form aufweisen. Als Wirkstoffe kommen Arzneistoffe aller Klassen in Betracht, beispielsweise Analgetika oder Psychopharmaka, oder auch Nikotin zur Raucherentwöhnung. Der im wirkstoffhaltigen Film enthaltene Wirkstoff wird nach der Verabreichung an einen Patienten aus dem Film freigesetzt und kann danach resorbiert werden.

Aufgrund des Wirkstoffgehalts der wirkstoffhaltigen Filme ist es wünschenswert oder sogar zwingend erforderlich, diese in einer solchen Weise zu verpacken, daß sie nicht von Unbefugten, insbesondere von Kindern, entnommen und eingenommen oder verschluckt werden können. Zumindest sollte der Öffnungsvorgang erschwert oder verzögert werden; andererseits soll eine solche kindersichere Verpackung dennoch für Erwachsene ohne größere Mühen und ohne Zuhilfenahme von Werkzeugen zu öffnen sein.

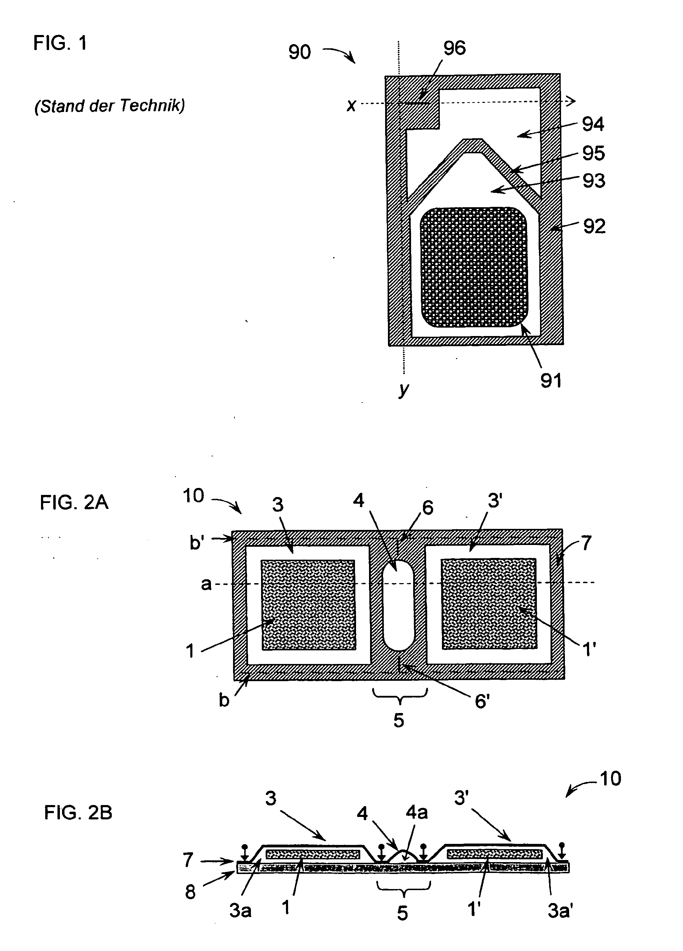

Bei einem bereits bekannten Produkt (Theraflu^{®}, Novartis AG) ist ein wirkstoffhaltiger Filmstreifen zwischen einer Träger- und einer Deckschicht eingeschlossen, die durch Siegelnähte miteinander verbunden sind. Eine derartige Verpackung (90) ist in Fig. 1 in Draufsicht schematisch dargestellt. Die beiden Packstoffbahnen (Träger- und Deckschicht) sind im Bereich der schraffierten Flächen miteinander versiegelt. Die Verpackung weist einen äußeren Siegelrand (92) auf, sowie einen Steg (95), der den Innenraum des so gebildeten Beutels in zwei Kompartimente (93, 94) unterteilt. Der erste Raum (93) enthält den verpackten, wirkstoffhaltigen Filmstreifen (91), der zweite Raum (94) ist leer und dient der Ausbildung einer kindersicheren Aufreißhilfe. Zu diesem Zweck ist die Verpackung in einem Bereich des gesiegelten Außenrandes mit einer Perforation oder Stanzung (96) versehen, die vom Außenrand beabstandet ist. Zum Öffnen der Verpackung muß diese entlang der Linie (y) geknickt werden, wodurch das Einreißen der Verpackung entlang der Linie (x) ermöglicht wird, die durch den Flächenbereich des zweiten Raumes (94) verläuft. Durch das Einreißen bzw. Abreißen werden frei zugängliche Kanten der Träger- und Deckschicht gebildet, die als Anfaßhilfe dienen und das Abziehen der Deckschicht von der Trägerschicht ermöglichen, wodurch der Verpackungsinhalt (91) zugänglich wird. Die konstruktionsbedingte Erschwerung des Öffnungsvorgangs trägt wesentlich zur Kindersicherheit der Verpackung bei.

Allerdings weist die vorstehend beschriebene bekannte Verpackung auch erhebliche Nachteile auf. Da die für die Ausbildung der Aufreißhilfe (94) benötigte Fläche relativ groß ist und ca. 30 % der Fläche des für die Aufnahme des Filmstreifens bestimmten Kompartiments (93) beträgt, ist der Materialaufwand - bezogen auf die Größe des zu verpackenden Filmstreifens - zu groß und erhöht die Produktionskosten. Die zum Einschließen des Packguts nutzbare Fläche (93) ist - bezogen auf die Gesamtfläche der Verpackung - zu niedrig und liegt bei ca. 40 %. Dies bedeutet nicht nur einen erhöhten Materialverbrauch bei der Herstellung, sondern auch einen erhöhten Platzbedarf bei der Lagerung.

Hinzu kommt, daß bei der vorstehend beschriebenen Verpackung die Siegelnähte, durch welche Träger- und Deckschicht miteinander lösbar verbunden sind, einen beträchtlichen Anteil an der Gesamtfläche der Verpackung haben, nämlich ca. 30 %. Infolgedessen muß die Produktionsgeschwindigkeit entsprechen reduziert werden, was wiederum eine Steigerung der Produktionskosten zur Folge hat.

DE 101 02 818 A1 beschreibt primär Verpackungseinheiten für mehrere vereinzelte wirkstoffhaltige Filme, die in dieser in mehreren in Reihen ausgebildeten Fächern angeordnet sind. Dieses Dokument offenbart die Merkmale der Oberbegriffe der Patentansprüche 1 und 12.

DE 10 2004 047 445 A1 offenbart nicht wiederverschließbare Verpackungen für gesundheitsgefährdende Erzeugnisse, wie Arzneimittel, die insbesondere als kindersichere Verpackung ausgebildet sind.

Die Aufgabe der vorliegenden Erfindung bestand deshalb darin, eine Verpackung für wirkstoffhaltige Filme sowie ein hierfür geeignetes Herstellungsverfahren bereitzustellen, wobei diese Verpackung ein kindersicheres Verpacken von wirkstoffhaltigen Filmen ermöglichen soll, jedoch unter Vermeidung oder Verminderung der vorstehend genannten Nachteile. Insbesondere bestand die Aufgabe darin, den Materialeinsatz zu verringern, die Produktionsgeschwindigkeit zu erhöhen, und eine bessere Flächenausnutzung der Verpackung zu ermöglichen. Erfindungsgemäß wird diese Aufgabe gelöst durch eine Verpackung mit den Merkmalen des Anspruchs 1.

Durch die paarweise Anordnung zweier gegenüberliegender Räume für die Aufnahme der wirkstoffhaltigen Filmstreifen wird eine optimale Ausnutzung der Gesamtfläche der Verpackung und damit eine Verringerung des Materialverbrauchs bewirkt. Aufgrund der Anordnung des die Aufreißhilfe bildenden weiteren Flächenbereichs im Bereich des Steges der Doppelverpackung verringert sich die hierfür benötigte Fläche relativ zu den Flächenbereichen, welche für die Unterbringung des Verpackungsguts bestimmt sind. Die erfindungsgemäße Anordnung der genannten Flächenbereiche ermöglicht es ferner, die Siegelnähte, welche im Bereich des Steges die Deckschicht mit der Trägerschicht verbinden, wesentlich schmaler auszubilden als die umlaufende Siegelnaht am Außenrand der Verpackung. Insgesamt wird dadurch eine Verringerung der durch Siegelung zu verbindenden Flächen erzielt, so daß höhere Produktionsgeschwindigkeiten ermöglicht werden. Zusätzlich weist auch die erfindungsgemäße Verpackung Merkmale auf, die - in ähnlicher Weise wie bei dem oben beschriebenen bekannten Produkt - das kindersichere Verpacken von wirkstoffhaltigen Produkten ermöglichen.

Durch die paarweise Verpackung von wirkstoffhaltigen Filmen kann bei vorgegebener Dauer des Siegelvorgangs die doppelte Anzahl von wirkstoffhaltigen Filmen verpackt werden. Hieraus resultiert eine zusätzliche Erhöhung der Produktionsgeschwindigkeit.

Die erfindungsgemäßen Verpackungen eignen sich insbesondere zum paarweisen Verpacken von solchen wirkstoffhaltigen Filmen, die aufgrund der Art oder der Dosis des/der enthaltenen Wirkstoffe(s) in der Regel gleichzeitig oder in kurzem zeitlichen Abstand nacheinander aus der Verpackung entnommen werden. Diese Situation ist beispielsweise im Falle von Analgetika häufig gegeben, wenn aufgrund der Stärke der Schmerzen die Verabreichung einer doppelten Dosis erforderlich ist. Des weiteren können die erfindungsgemäßen Verpackungen insbesondere zum paarweisen Verpacken von Filmen verwendet werden, die unterschiedliche Wirkstoffe enthalten und die zwecks Verabreichung einer Wirkstoffkombination jeweils paarweise aus der Verpackung entnommen werden.

Für die Verstellung der Trägerschicht und der Deckschicht kommen Packstoffe aus Papier, Pappe, Kunststoff-Folien (z. B. Polyethylen, Polyethylenterephthalat, Polypropylen, Polystyrol, Cellophan, Polyamide, Polycarbonate, EthylenVinylacetat-Copolymer) und Metallfolien (z. B. Aluminiumfolie) sowie Verbundmaterialien aus den genannten Materialien in Betracht. Ein weiteres, bevorzugtes Folienmaterial ist Barex^{®} (BP Chemicals), ein Copolymer aus Acrylnitril und Butadien. Aufgrund seiner guten Barriere-Eigenschaften und chemischen Widerstandsfähigkeit eignet es sich insbesondere zum Verpacken von Arzneimitteln mit einem Gehalt an aggressiven und/oder flüchtigen Wirkstoffen, z. B. Nicotin.

Um eine unbefugte oder versehentliche Zerstörung der Verpackung durch Zerreißen, spitze Gegenstände etc. auszuschließen, ist es erforderlich, daß jede der beiden Packstoffkomponenten (Trägerschicht, Deckschicht) eine ausreichende Reißfestigkeit aufweist (z. B. nach DIN 53455 oder EN-ISO 527). Hierfür geeignete Materialien sind dem Fachmann bekannt. Die Dicke der Trägerschicht und der Deckschicht liegt vorzugsweise im Bereich von 0,01 bis 2 mm, insbesondere 0,05 bis 0,5 mm. In einer bevorzugten Ausführungsform der Erfindung weist die Trägerschicht der Verpackung eine höhere Dicke auf als die Deckschicht.

Trägerschicht und Deckschicht können aus denselben Materialien hergestellt sein, oder aus verschiedenartigen Materialien. Vorzugsweise besteht mindestens einer der beiden Packstoffanteile (Trägerschicht, Deckschicht) aus transparentem Material (z. B. transparente Kunststofffolie). Des weiteren umfaßt die Erfindung Ausführungsformen, bei denen ein Packstoffanteil oder beide Packstoffanteile gleich oder unterschiedlich gefärbt sind, wobei es sich jeweils um eine transparente oder opake Färbung handeln kann.

Beispielsweise kann die Trägerbahn aus einem nichttransparenten Verbundwerkstoff aus Papier (oder Pappe) mit Kunststoffen (z. B. mit Polyethylen oder Polyethylenterephthalat beschichtete Papiere), und die Deckschicht aus einer transparenten, farblosen oder gefärbten Kunststofffolie hergestellt sein. Zur Verminderung der Luft-, Licht- und Wasserdampfdurchlässigkeit ist es vorteilhaft, wenn zumindest eine Oberfläche der Trägerschicht oder/und der Deckschicht metallisiert ist (z. B. mit Aluminium beschichtet).

Die erfindungsgemäße Verpackung kann in den verschiedenartigsten geometrischen Formen (z. B. Rechteck, Trapez, Ellipse) und in unterschiedlichen Abmessungen hergestellt werden. Die Flächenausdehnung ist im allgemeinen abhängig von der Größe des Packguts (z. B. Wafer) und liegt üblicherweise im Bereich von 10 bis 100 cm². Ebenso kann die Größe des ersten Flächenbereichs zur Aufnahme des Packguts in weiten Bereichen variiert werden, abhängig von der Flächenausdehnung der zu verpackenden Objekte.

Trägerbahn und Deckschicht können identische Form und Größe haben; es sind aber auch Ausführungsformen vorgesehen, bei denen die Deckschicht eine geringere Größe als die Trägerschlicht oder/und eine von der Trägerschicht abweichende geometrische Form hat.

Die Trägerschicht ist mit der Deckschicht lösbar verbunden, und zwar so, daß die Deckschicht mittels Fingerkraft und ohne Zuhilfenahme von Werkzeugen von der Trägerschicht abgezogen werden kann, wie dies von anderen peelfähigen Verpackungen bekannt ist.

Die lösbare Verbindung zwischen Deckschicht und Trägerschicht wird vorzugsweise durch Siegeln oder Schweißen erzeugt; geeignete Mittel und Verfahren zum Erzeugen von Siegelnähten oder Siegelflächen sind dem Fachmann bekannt. Die Siegelung dient als Diffusions- und Permeationsbarriere und ist im allgemeinen undurchlässig für Wirkstoffe und Luftfeuchtigkeit. Es kommen sowohl Heißsiegelverfahren als auch Kaltsiegelverfahren in Betracht. Als Material für Siegelschichten können z. B. Schmelzkleber (Hotmelts; z.B. auf Basis von Polyethylen-LD), Siegellacke, Siegeldispersionen oder Klebstoffe verwendet werden.

Vorzugsweise wird die lösbare Verbindung durch Heißsiegeln bei Temperaturen im Bereich zwischen 50 °C und 200 °C, insbesondere 50 bis 90 °C, unter Verwendung von Hotmelts erzeugt.

Die Siegelnähte oder Siegelflächen weisen vorzugsweise eine Festigkeit (= Siegelfestigkeit) im Bereich von 1 N/15 mm bis 50 N/15 mm, vorzugsweise 2 N/15 mm bis 20 N/15 mm, auf.

Träger- und Deckschicht sind vorzugsweise vollflächig miteinander verbunden, mit Ausnahme der genannten Flächenbereiche. Im Bereich des Stegs sind die beiden Packstoffbahnen (Träger-, Deckschicht) miteinander versiegelt, mit Ausnahme desjenigen Bereichs, der die Aufreißhilfe bildet. Der Steg verläuft im wesentlichen senkrecht zur Längsrichtung der Verpackung.

Wie erwähnt, wird durch die erfindungsgemäße Konstruktionsweise eine besonders günstige Nutzung der Gesamtfläche der Verpackung ermöglicht. Gemäß einer bevorzugten Ausführungsform der Erfindung werden die genannten Flächenbereiche so gewählt, daß die Summe der Flächen der zwei gegenüberliegenden Flächenbereiche, welche die Räume zur Aufnahme der genannten Filme bilden, mindestens 50 %, vorzugsweise mindestens 60 %, der Gesamtfläche der Verpackung entspricht. Ferner wird bevorzugt, daß die Fläche des genannten weiteren Flächenbereichs nicht mehr als 50 %, vorzugsweise nicht mehr alls 20 %, besonders bevorzugt nicht mehr als 15 %, der Summe der Flächen der zwei gegenüberliegenden Flächenbereiche, welche die Räume zur Aufnahme der genannten Filme bilden, beträgt.

Die zwei paarweise angeordneten, gegenüberliegenden Flächenbereiche, welche die Räume zur Aufnahme der genannten Filme bilden, haben bevorzugt dieselbe Größe und dieselbe geometrische Form. Für bestimmte Anwendungen kann es allerdings vorteilhaft sein, diese beiden Flächenbereiche unterschiedlich zu gestalten.

Im Hinblick auf eine möglichst effiziente Fertigung ist es besonders vorteilhaft, wenn die zwei gegenüberliegenden Flächenbereiche, welche die Räume zur Aufnahme der genannten Filme bilden, dieselbe Größe und dieselbe geometrische Form aufweisen und in Bezug auf die Längsrichtung der Verpackung symmetrisch angeordnet sind, und wenn die Breite des zwischen den beiden Flächenbereichen befindlichen Steges der doppelten Breite des Randes entspricht, der zwischen einem stirnseitigen Außenrand der Verpackung und dem angrenzenden Flächenbereich, der zur Aufnahme des genannten Filmes bestimmt ist, verläuft. Dies hat zur Folge, daß bei der Fertigung ein nahezu gleichmäßiger Abstand zwischen den jeweils benachbarten wirkstoffhaltigen Filmen (bezogen auf die Längsrichtung der Verpackungen) eingehalten werden kann. Dadurch gestaltet sich der Herstellungsprozeß wesentlich einfacher und es werden höhere Prozeßgeschwindigkeiten ermöglicht. Außerdem wird dadurch eine im wesentlichen gleichmäßige Produktspreizung in Längsrichtung möglich. Bei Bedarf kann der Abstand zwischen den jeweils benachbarten wirkstoffhaltigen Filmen auch exakt gleichmäßig eingestellt werden, woraus eine maximale Fertigungsgeschwindigkeit resultiert.

Es ist erfindungswesentlich, dass die genannten Flächenbereiche so bemessen und derartig angeordnet sind, daß die Summe der Flächenbereiche, in denen die Deckschicht mit der Trägerschicht verbunden ist, nicht mehr als 25 %, vorzugsweise nicht mehr als 20 % beträgt. Auf diese Weise läßt sich die für den Siegelvorgang benötigte Zeit reduzieren. Die Produktstabilität der verpackten Filme wird durch die am Außenrand der Verpackung verlaufende Siegelnaht gewährleistet. Deren Breite beträgt vorzugsweise mindestens 3 mm, insbesondere mindestens 5 mm. Für die im Bereich des Steges verlaufenden Siegelränder, welche den ersten bzw. zweiten Flächenbereich von dem genannten weiteren Flächenbereich trennen, ist eine Breite von 1,5 bis 2 mm ausreichend, um den Schutz des Packguts zu gewährleisten.

Nach einer bevorzugten Ausführungsform ist die erfindungsgemäße Verpackung derart aufgebaut, daß zwischen einem jeden der beiden Flächenbereiche, welche die allseitig umschlossenen Räume zur Aufnahme der genannten Filme bilden, und dem jeweils benachbarten Außenrand der Verpackung ein Bereich vorhanden ist, innerhalb dessen die Trägerschicht mit der Deckschicht verbunden ist und der eine Breite von 3 bis 7 mm, vorzugsweise 4 - 5 mm aufweist.

Zur Ausbildung einer Aufreißhilfe ist bei den erfindungsgemäßen Verpackungen innerhalb des genannten Stegs ein weiterer Flächenbereich vorhanden, in welchem die Trägerschicht nicht mit der Deckschicht verbunden ist, wodurch ein allseitig umschlossener Hohlraum gebildet wird. Dieser dient lediglich der Ausbildung einer Anfaß- und Aufreißhilfe, und ist nicht für die Aufnahme von wirkstoffhaltigen Filmen bestimmt.

Der genannte weitere Flächenbereich weist vorzugsweise eine längliche Form auf. Nach einer bevorzugten Ausführungsform ist der genannte weitere Flächenbereich mit einer wellenförmigen oder sägezahnförmigen Kontur versehen, wodurch der Peel- oder Abziehvorgang erleichtert wird. Dies ist insbesondere dann von Vorteil, wenn die nach dem Einreißen der Perforation gebildete Anfaßhilfe relativ kurz ist.

Wie erwähnt, ist innerhalb des Stegs mindestens eine Perforationslinie vorhanden. Diese ist vorzugsweise so ausgebildet, daß sie in Richtung auf den genannten weiteren Flächenbereich hin verläuft. Durch Einreißen der Träger- und Deckschicht an der Perforationslinie und anschließendes Weiterreißen wird der erwähnte zweite Flächenbereich annähernd in zwei Hälften zerteilt. Durch das Durchtrennen der beiden Schichten der Verpackung entstehen freie Kanten, die als Aufreißhilfe dienen können, wie vorstehend beschrieben. Auf diese Weise wird beim Einreißen der Verpackung für jede der beiden Räume, in denen die wirkstoffhaltigen Filme eingeschlossen sind, eine Aufreißhilfe erzeugt.

Die erwähnte Perforationslinie kann auf bekannte Weise, z.B. durch Stanzen, erzeugt werden. Neben solchen Perforationslinien kommen auch andere Arten von Schwächungslinien in Betracht, sofern sie das Einreißen der Verpackung ermöglichen. Die Perforationslinie wird vorzugsweise so ausgebildet, daß die zum Durchtrennen erforderliche Fingerkraft von erwachsenen Personen leicht, im allgemeinen aber nicht von Kleinkindern aufgebracht werden kann.

Die Kindersicherheit wird dadurch erreicht, daß zum Öffnen der Verpackung mindestens drei koordinierte manuelle Vorgänge erforderlich sind: (i) Knicken der Verpackung im Randbereich, um die Perforationslinie zugänglich zu machen; (ii) Einreißen und Weiterreißen der Perforation, und (iii) Ergreifen der so erzeugten freien Kanten als Anfaßhilfe (oder Anfaßlasche), und Auseinanderziehen von Träger- und Deckschicht.

Als Strukturen, welche das Einreißen des/der Packstoffelemente ermöglichen, eignen sich insbesondere: gerade Schnitte; gezackte oder wellenförmige Schnitte; Perforationen, insbesondere Perforationen aus hintereinander angeordneten Punkten oder/und Schnitten; Materialaussparungen; Stanzungen, insbesondere pfeilförmige, dreieckige oder rautenförmige Stanzungen; Sollbruchstellen oder geprägte Linien.

Um den erwähnten Vorgang des Faltens oder Knickens der Verpackung zu erleichtern, ist es vorteilhaft, die Verpackung mit einer Falzlinie oder Knicklinie zu versehen, beispielsweise durch Prägung oder Quetschung. Hierfür geeignete Methoden sind dem Fachmann bekannt.

Die genannte Falzlinie oder Knicklinie ist vorzugsweise so angeordnet, daß sie in Längsrichtung der Verpackung verläuft und die erwähnte Perforationslinie berührt oder schneidet, beispielsweise rechtwinklig. Auf diese Weise wird der Vorgang des Einreißens erheblich erleichtert, ohne daß die Kindersicherheit der Verpackung beeinträchtigt wird.

Dies ist insbesondere im Hinblick auf den bestimmungsgemäßen Gebrauch der Verpackung durch ältere Personen von Bedeutung, da auch in diesem Fall gewährleistet werden muß, daß die Verpackung ohne Zuhilfenahme von Werkzeugen oder sonstigen Hilfsmitteln geöffnet werden kann, und daß das Öffnen innerhalb kurzer Zeit und ohne frustrierende Versuche möglich ist.

Die Perforationslinie (n) oder zumindest eine der Perforationslinien ist/sind vorzugsweise sowohl in der Trägerschicht als auch in der Deckschicht angebracht, um ein einfaches und sicheres Einreißen zu ermöglichen.

Bezüglich der Position der Perforationslinien wird bevorzugt, daß die Perforationslinie (n) oder zumindest eine der Perforationslinien in dem Bereich angeordnet ist/sind, der sich zwischen dem Außenrand der Verpackung und dem genannten weiteren Flächenbereich befindet. Um die gewünschte Kindersicherheit, zu gewährleisten, reicht (reichen) die Perforationslinie (n) erfindungsgemäß nicht bis an den Außenrand der Verpackung heran.

Gemäß einer bevorzugten Ausführungsform der Erfindung ist vorgesehen, daß die Perforationslinie(n) oder zumindest eine der Perforationslinien nicht bis an den genannten weiteren Flächenbereich, der für die Ausbildung der Aufreißhilfe bestimmt ist, heranreicht bzw. heranreichen.

Aufgrund der oben beschriebenen Ronstruktionsmerkanale ist die erfindungsgemäße Verpackung zum kindersicheren Verpacken von wirkstoffhaltigen Filmen geeignet. Die Erfindung umfaßt somit kindergesicherte, nicht wiederverschließbare Verpackungen mit den vorstehend beschriebenen Merkmalen. Insbesondere umfaßt die vorliegende Erfindung Verpackungen der vorstehend beschriebenen Art, die sich dadurch auszeichnen, daß sie kindersicher gemäß DIN EN 14375 oder/und nach ASTM D3475-03a sind.

Die erfindungsgemäßen Verpackungen eignen sich zum Verpacken, insbesondere zum kindersicheren Verpacken von wirkstoffhaltigen Filmen der eingangs beschriebenen Art. Als Wirkstoffe kommen neben den erwähnten pharmazeutischen Wirkstoffe auch kosmetische Wirkstoffe, Geschmacks- und Aromastoffe, Nährstoffe, Vitamine, diagnostische Reagenzien, Giftstoffe und Wirkstoffe, die im Pflanzenschutz oder zur Schädlingsbekämpfung eingesetzt werden, in Betracht.

Mit der Erfindung wird ferner ein Verfahren zum Verpacken von wirkstoffhaltigen Filmen bereitgestellt, wobei das Verfahren die Merkmale des Anspruchs 12 aufweist.

Nach einer bevorzugten Ausführungsform wird das Verfahren in der Weise durchgeführt, daß die zwei gegenüberliegenden Flächenbereiche, welche die Räume zur Aufnahme der genannten Filme bilden, dieselbe Größe und dieselbe geometrische Form aufweisen und symmetrisch angeordnet werden. Ferner wird der zwischen den beiden Flächenbereichen befindliche Steg in der Weise ausgebildet, daß seine Breite der doppelten Breite desjenigen Randes entspricht, der zwischen einem stirnseitigen Außenrand der Verpackung und dem angrenzenden Flächenbereich, der zur Aufnahme des genannten Filmes bestimmt ist, verläuft.

Im Folgenden werden die Merkmale, Einzelheiten und Vorteile der Erfindung anhand der in den Zeichnungen schematisch dargestellten Ausführungsformen beispielhaft erläutert. Die Darstellungen in den Zeichnungen geben nicht notwendigerweise die tatsächlichen Größenverhältnisse wieder.
FIG. 2A zeigt eine erfindungsgemäße Verpackung (10) in Draufsicht.
FIG. 2B zeigt einen Schnitt in der Ebene (a) der FIG. 2A.
FIG. 3A, 3B, 3C und 3D zeigen weitere Ausführungsformen der erfindungsgemäßen Verpackung in Draufsicht.
FIG. 4 zeigt - ebenfalls in Draufsicht - zwei in Längsrichtung hintereinander angeordnete Verpackungen im Zustand während der Herstellung.

FIG. 2A zeigt eine Verpackung (10) mit einem rechteckigen Grundriß, die aus einer Deckschicht (7) und einer darunter liegenden, mit der Deckschicht verbundenen Trägerschicht aufgebaut ist (siehe Fig. 2B). Die Deckschicht ist in FIG. 2A transparent dargestellt.

Die Verpackung weist einen ersten Flächenbereich (3) und einen zweiten Flächenbereich (3') auf, innerhalb welcher die Deckschicht nicht mit der Trägerschicht verbunden ist. Innerhalb der Flächenbereiche (3, 3') befinden sich die verpackten wirkstoffhaltigen Filme (1, 1'). Die Deckschicht (7) hat dieselbe geometrische Form und Größe wie die darunter liegende Trägerschicht und ist mit dieser vollflächig und lösbar, d. h. peelfähig, verbunden (schraffierte Bereiche), mit Ausnahme des ersten und zweiten Flächenbereichs (3, 3').

Die beiden Flächenbereiche (3, 3') sind vollständig von einem Rand umgeben, in dem die Deckfolie (7) mit der Trägerschicht verbunden ist. Zwischen dem ersten Flächenbereich (3) und dem zweiten Flächenbereich (3') befindet sich ein Steg (5), in dessen Bereich die Deckfolie ebenfalls mit der Trägerschicht verbunden ist. Innerhalb des Stegs (5), und jeweils beabstandet von den Flächenbereichen (3, 3') befindet sich ein weiterer Flächenbereich (4), innerhalb dessen die Deckfolie (7) nicht mit der Trägerfolie verbunden ist, und der zur Ausbildung einer Anfaß- und Aufreißhilfe dient. Im Bereich des Stegs (5) sind zwei Schwächungslinien in Form von Perforationslinien (6, 6') angebracht, und zwar beabstandet vom Außenrand und vom weiteren Flächenbereich (4). Die Perforationslinien verlaufen senkrecht zur Längsrichtung der Verpackung (Linie a), ebenso wie der längliche Flächenbereich (4), und sind in Bezug auf diesen Flächenbereich mittig angeordnet.

Die Verpackung weist ferner eine in Längsrichtung verlaufende Knicklinie (b) auf, die in einem seitlichen Randbereich verläuft und die Perforationslinie (6') berührt. Optional kann eine zweite Knicklinie (b') vorgesehen sein. Nach Umknicken des Außenrandes der Verpackung längs der Knicklinie (b oder b') kann die Verpackung an einer der Perforationslinien (6, 6') eingerissen werden und die Deck- und Trägerfolie im Bereich des Flächenbereichs (4) in zwei annähernd gleich große Hälften zerteilt werden. Die durch den Riß gebildeten freien Kanten der Deck- und Trägerschicht können nun als Anfaß- und Aufreißhilfen verwendet werden, um die beiden Schichten voneinander abzulösen und die Entnahme der wirkstoffhaltigen Filme (1, 1') zu ermöglichen.

Beispielsweise hat die in FIG. 2A gezeigte Verpackung eine Länge von 96 mm und eine Breite von 42 mm. Die den ersten bzw. zweiten Flächenbereich umgebende Siegelnaht ist 5 mm breit, so daß jeder dieser Flächenbereiche eine Größe von 32 x 32 mm hat. Die zu verpackenden wirkstoffhaltigen Filme (1, 1') haben bei diesem Beispiel eine Größe von ca. 22 x 22 mm. Der im Bereich des Stegs liegende Flächenbereich (4) hat - in Bezug auf seine langgestreckte Form - eine Breite von 10 mm und ist von den benachbarten Flächenbereichen (3, 3') durch eine 5 mm breite Siegelnaht getrennt, so daß die Gesamtbreite des Stegs bei diesem Beispiel 20 mm beträgt. Beim Öffnen der Verpackung wird aus dem Flächenbereich (4) für jeden der beiden verpackten Filme eine Anfaßhilfe von ca. 5 bis 5,5 mm Länge (bezogen auf die Längsrichtung der Verpackung) gebildet.

FIG. 2B zeigt einen Schnitt in der Ebene (a) der FIG. 2A. Die Verpackung ist aus einer Trägerschicht (8) und einer Deckschicht (7) aufgebaut. Die beiden Schichten sind in den mit Pfeilen bezeichneten Bereichen lösbar miteinander verbunden. Im Bereich der Flächenbereiche (3, 3') sind die beiden Schichten nicht miteinander verbunden und bilden Räume (3a, 3a') zur Aufnahme der wirkstoffhaltigen Filme (1, 1'). Im Bereich des Flächenbereichs (4) wird durch die nicht miteinander verbundenen Schichten ein Hohlraum (4a) ausgebildet.

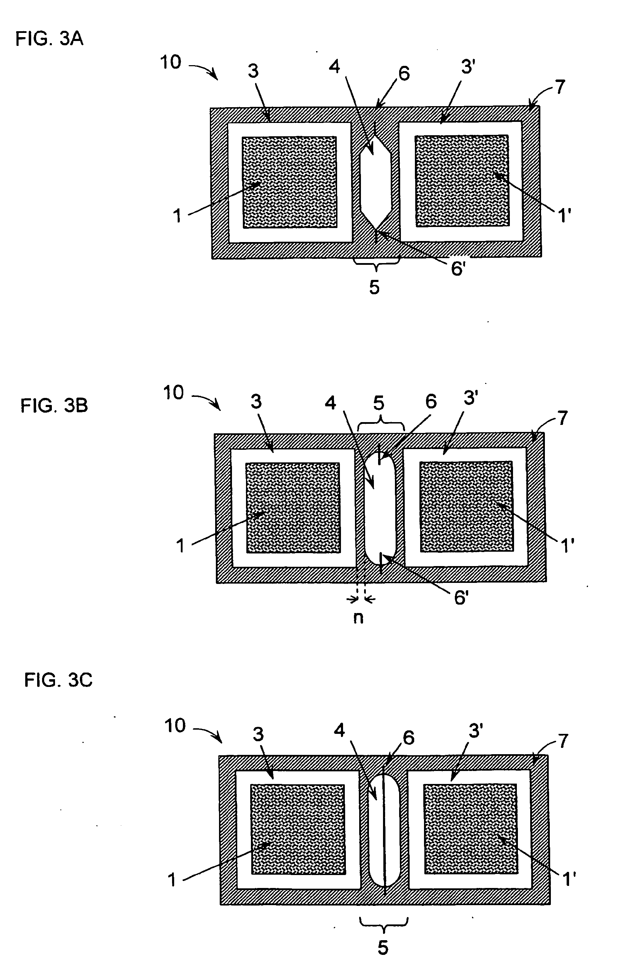

FIG. 3A zeigt eine Abwandlung der in Fig. 1A gezeigten Verpackung, wobei der weitere Flächenbereich (4) die Form eines langgestreckten Polygons hat und die beiden gegenüberliegenden Perforationen (6, 6'), die annähernd senkrecht zur Längsrichtung der Verpackung verlaufen, bis an diesen Flächenbereich (4) heranreichen.

FIG. 3B zeigt eine weitere Abwandlung der in Fig. 1A gezeigten Verpackung, wobei die Perforationen (6, 6') bis in den Innenraum des weiteren Flächenbereichs (4) verlängert sind.

Die Breite (n) der Siegelnaht, welche den Flächenbereich (4) gegen die Flächenbereiche (3, 3') abgrenzt, kann geringer sein als die Breite der Siegelnähte, welche die Flächenbereiche (3, 3') zum Außenrand der Verpackung hin abschließen. Ausgehend von den bei FIG. 2A beispielhaft genannten Maßangaben kann die Breite (n) beispielsweise 2 mm betragen. Dies ermöglicht es, die Breite des Flächenbereichs (4) (in Bezug auf dessen Längsrichtung) zu vergrößern (z. B. 25 mm), so daß die daraus hervorgehenden Anfaßhilfen größer und besser zu greifen sind.

FIG. 3C zeigt eine weitere Abwandlung der in Fig. 1A gezeigten Verpackung, wobei die Perforationslinie (6) vollständig durch den Innenraum des weiteren Flächenbereichs (4) hindurch verläuft.

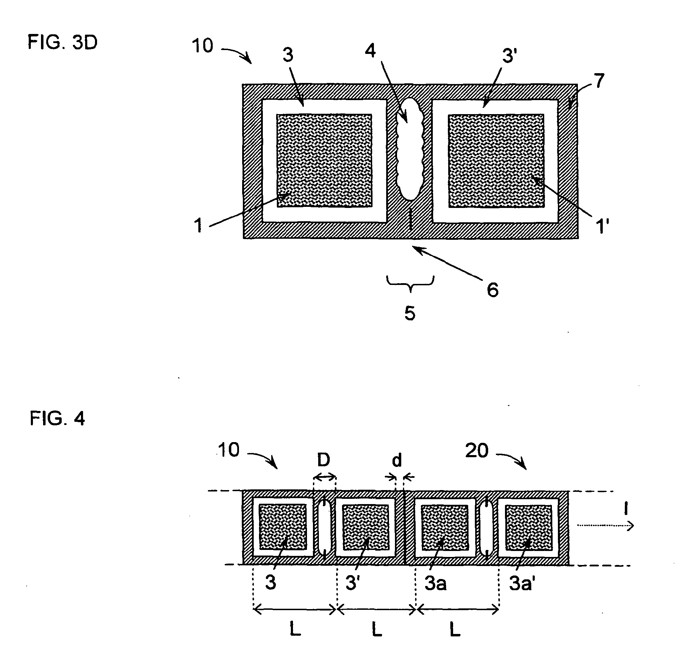

FIG. 3D zeigt eine weitere Abwandlung der in Fig. 1A gezeigten Verpackung, wobei der im Bereich des Stegs (5) liegende Flächenbereich (4) eine gezackte oder wellenförmige Kontur aufweist. Die im Stegbereich angebrachte Perforationslinie (6) befindet sich im Bereich zwischen der Außenkante der Verpackung und dem Flächenbereich (4).

FIG. 4 zeigt zwei aufeinander folgende Verpackungen (10, 20) der in FIG. 3B gezeigten Art während der Herstellung. Die zwei gegenüberliegenden Flächenbereiche, welche die Räume zur Aufnahme der Filme (3, 3'; 3a, 3a') bilden, haben im wesentlichen dieselbe Größe und dieselbe geometrische Form und sind in Bezug auf die Längsrichtung (Pfeil 1) symmetrisch angeordnet. Der zwischen den beiden Flächenbereichen befindliche Steg ist in der Weise ausgebildet, daß seine Breite D der doppelten Breite d des Randes entspricht, der zwischen einem stirnseitigen Außenrand der Verpackung und dem angrenzenden Flächenbereich, der zur Aufnahme des genannten Filmes bestimmt ist, verläuft. Dadurch wird erreicht, daß die wirkstoffhaltigen Filme (3, 3', 3a, 3a') mit im wesentlichen gleichbleibendem Abstand L verpackt werden können.

Beispielsweise hat jede der in FIG. 4 gezeigten Verpackungen eine Länge von 88 mm und eine Breite von 42 mm. Die den ersten bzw. zweiten Flächenbereich umgebende Siegelnaht ist zu den Außenrändern der Verpackung hin 5 mm breit. Die Breite der Siegelnaht, welche im Bereich des Stegs (5) den Flächenbereich (4) gegen die Flächenbereiche (3, 3') abgrenzt, ist geringer als die Breite der Siegelnähte, welche die Flächenbereiche (3, 3') zum Außenrand der Verpackung hin abschließen, und beträgt 1,5 bis 2 mm. Der langgestreckte Flächenbereich (4) hat eine Breite von ca. 10 mm, so daß beim Öffnen der Verpackung für jeden der beiden verpackten Filme eine Anfaßhilfe von ca. 5 bis 5,5 mm Länge (bezogen auf die Längsrichtung der Verpackung) gebildet wird. Vorteilhafterweise ist der Flächenbereich (4) wie in FIG. 3D geformt.

Jeder der Flächenbereiche (3, 3') hat eine Größe von ca. 3 x 3 cm, und die zu verpackenden wirkstoffhaltigen Filme (1, 1') haben bei diesem Beispiel eine Größe von ca. 22 x 22 mm. Bei der beschriebenen Anordnung der Flächenbereiche und der Siegelnähte beträgt die durch Diffusion zu überwindende Siegelnahtbreite mindestens 5 mm zwischen den Innenräumen der Verpackung und der Umgebung, so daß die Produktstabilität gewährleistet ist, solange die Verpackung ungeöffnet ist.

Die Erfindung ermöglicht in vorteilhafter Weise die Verringerung des Materialverbrauchs und die Erhöhung der Produktionsgeschwindigkeit bei der Herstellung von kindersicheren Verpackungen für wirkstoffhaltige Filme.

## Patentansprüche

1. Verpackung (10) für wirkstoffhaltige Filme (1, 1'), die eine Trägerschicht (8) und eine mit dieser lösbar verbundene Deckschicht (7) aufweist, wobei
- die Verpackung in einer paarweisen Anordnung zwei gegenüberliegende, durch einen Steg (5) voneinander getrennte Flächenbereiche (3, 3') aufweist, innerhalb welcher die Deckschicht (7) nicht mit der Trägerschicht (8) verbunden ist, wodurch zwei voneinander getrennte, allseitig umschlossene Räume zur paarweisen Aufnahme der genannten Filme (1, 1') gebildet werden;
- innerhalb des genannten Stegs (5) ein weiterer Flächenbereich (4) vorhanden ist, in welchem die Trägerschicht (8) nicht mit der Deckschicht (7) verbunden ist, wodurch ein allseitig umschlossener Hohlraum gebildet wird;
- innerhalb des Stegs (5) mindestens eine Perforationslinie (6) vorhanden ist, **dadurch gekennzeichnet, dass** die Perforationslinie (6) nicht bis an den Außenrand der Verpackung heranreicht und die Summe der Flächenbereiche, in denen die Deckschicht (7) mit der Trägerschicht (8) verbunden ist, nicht mehr als 25 % beträgt.

2. Verpackung nach Anspruch 1, **dadurch gekennzeichnet, daß** die Perforationslinie(n) oder zumindest eine der Perforationslinien (6, 6') sowohl in der Trägerschicht (8) als auch in der Deckschicht (7) angebracht ist/sind.

3. Verpackung nach Anspruch 1 oder 2, **dadurch gekennzeichnet, daß** die Perforationslinie (n) oder zumindest eine der Perforationslinien (6, 6') in dem zwischen dem Außenrand der Verpackung und dem genannten weiteren Flächenbereich (4) liegenden Bereich angeordnet ist/sind.

4. Verpackung nach einem der Ansprüche 1 bis 3, **dadurch gekennzeichnet, daß** die Perforationslinie(n) in Richtung auf den genannten weiteren Flächenbereich (4) hin verläuft bzw. verlaufen.

5. Verpackung nach Anspruch 4, **dadurch gekennzeichnet, daß** die Perforationslinie(n) oder zumindest eine der Perforationslinien (6, 6') nicht bis an den genannten weiteren Flächenbereich (4) heranreicht bzw. heranreichen.

6. Verpackung nach Anspruch 4, **dadurch gekennzeichnet, daß** die Perforationslinie(n) oder zumindest eine der Perforationslinien (6, 6') bis an den genannten weiteren Flächenbereich (4) heranreicht oder sich in diesen hinein erstreckt.

7. Verpackung nach einem der vorangehenden Ansprüche, **dadurch gekennzeichnet, daß** die Trägerschicht (8) vollflächig mit der Deckschicht (7) verbunden ist, mit Ausnahme der genannten Flächenbereiche (3, 3'), und daß zwischen einem jeden der beiden Flächenbereiche (3, 3'), welche die allseitig umschlossenen Räume zur Aufnahme der genannten Filme bilden, und dem jeweils benachbarten Außenrand der Verpackung ein Bereich vorhanden ist, innerhalb dessen die Trägerschicht (8) mit der Deckschicht (7) verbunden ist und der eine Breite von 3 bis 7 mm, vorzugsweise 4 - 5 mm aufweist.

8. Verpackung nach einem der vorangehenden Ansprüche, **dadurch gekennzeichnet, daß** die zwei gegenüberliegenden Flächenbereiche (3, 3'), welche die Räume zur Aufnahme der genannten Filme bilden, dieselbe Größe und dieselbe geometrische Form aufweisen und in Bezug auf die Längsrichtung der Verpackung symmetrisch angeordnet sind, und daß die Breite des zwischen den beiden Flächenbereichen (3, 3') befindlichen Steges (5) der doppelten Breite des Randes entspricht, der zwischen einem stirnseitigen Außenrand der Verpackung und dem angrenzenden Flächenbereich, der zur Aufnahme des genannten Filmes bestimmt ist, verläuft.

9. Verpackung nach einem der vorangehenden Ansprüche, **dadurch gekennzeichnet, daß** die genannten lösbaren Verbindungen durch Siegelnähte oder Siegelflächen gebildet sind.

10. Verpackung nach einem der vorangehenden Ansprüche, **dadurch gekennzeichnet, daß** sie mindestens eine Falz- oder Knicklinie (b) aufweist, die in Längsrichtung der Verpackung verläuft und die genannte Perforation (6) berührt oder schneidet.

11. Verwendung einer Verpackung nach einem der vorangehenden Ansprüche zum Verpacken von wirkstoffhaltigen Filmen, vorzugsweise zum kindersicheren Verpacken von wirkstoffhaltigen Filmen.

12. Verfahren zum Verpacken von wirkstoffhaltigen Filmen, wobei das Verfahren folgende Schritte aufweist:
- Bereitstellen einer Trägerschicht (8);
- Positionieren zweier wirkstoffhaltiger Filme in paarweiser Anordnung auf der Trägerschicht (8);
- Bedecken der Trägerschicht (8) und der darauf befindlichen wirkstoffhaltigen Filme (1, 1') mit einer Deckschicht (7);
- Verbinden von Trägerschicht (8) und Deckschicht (7) mittels einer lösbaren Verbindung, und zwar in der Weise, daß die beiden Flächenbereiche (3, 3'), welche die wirkstoffhaltigen Filme (1, 1') einschließen, vollständig von einem Randbereich umgeben werden, in dem die Trägerschicht (8) mit der Deckschicht (7) verbunden ist, wodurch zwei allseitig umschlossene Räume gebildet werden, die die beiden Filme enthalten, sowie ein zwischen diesen Räumen liegender Steg (5), und daß das Verbinden von Trägerschicht (8) und Deckschicht (7) ferner in der Weise erfolgt, daß die Verpackung im Bereich des zwischen den beiden genannten Flächenbereichen (3, 3') vorhandenen Steges (5) einen weiteren Flächenbereich (4) aufweist, in welchem die Trägerschicht (8) nicht mit der Deckschicht (7) verbunden ist;
- Erzeugen mindestens einer Perforationslinie (6) innerhalb des Stegs, **dadurch gekennzeichnet, daß** die Perforationslinie (6) nicht bis an den Außenrand der Verpackung heranreicht und die Summe der Flächenbereiche, in denen die Deckschicht (7) mit der Trägerschicht (8) verbunden ist, nicht mehr als 25 % beträgt.

13. Verfahren nach Anspruch 12, **dadurch gekennzeichnet, daß** die zwei gegenüberliegenden Flächenbereiche (3, 3'), welche die Räume zur Aufnahme der genannten Filme bilden, dieselbe Größe und dieselbe geometrische Form aufweisen und in Bezug auf die Längsrichtung der herzustellenden Verpackung symmetrisch angeordnet werden, und daß der zwischen den beiden Flächenbereichen (3, 3') befindliche Steg (5) in der Weise ausgebildet wird, daß seine Breite der doppelten Breite des Randes entspricht, der zwischen einem stirnseitigen Außenrand der Verpackung und dem angrenzenden Flächenbereich, der zur Aufnahme des genannten Filmes bestimmt ist, verläuft.

14. Verfahren nach Anspruch 12 oder 13, **dadurch gekennzeichnet, daß** die Verbindung zwischen Trägerschicht (8) und Deckschicht (7) durch Siegelung erzeugt wird, insbesondere unter Verwendung eines peelfähigen Siegellackes.

15. Verfahren nach einem der Ansprüche 12 bis 14, **dadurch gekennzeichnet, daß** eine Verpackung mit den in den Ansprüchen 1 bis 10 beschriebenen Merkmalen hergestellt wird.

## Claims

1. A packaging (10) for active substance-containing films (1, 1'), which has a carrier layer (8) and a covering layer (7) releasably connected to the latter, wherein
- the packaging has in a paired arrangement two opposite surface regions (3, 3') which are separated from one another by a web (5) and within which the covering layer (7) is not connected to the carrier layer (8), with the result that two spaces, separate from one another and enclosed on all sides, for receiving said films (1, 1') in pairs are formed;
- within said web (5), a further surface region (4) is present, in which the carrier layer (8) is not connected to the covering layer (7), with the result that a cavity enclosed on all sides is formed;
- at least one perforation line (6) is present within the web (5), **characterised in that** the perforation line (6) does not extend as far as the outer margin of the packaging, and that the sum of the surface regions in which the covering layer (7) is connected to the carrier layer (8) amounts to no more than 25%.

2. The packaging as claimed in claim 1, **characterized in that** the perforation line or perforation lines or at least one of the perforation lines (6, 6') is/are formed both in the carrier layer (8) and in the covering layer (7).

3. The packaging as claimed in claim 1 or 2, **characterized in that** the perforation line or perforation lines or at least one of the perforation lines (6, 6') is/are arranged in the region lying between the outer margin of the packaging and said further surface region (4).

4. The packaging as claimed in any one of claims 1 to 3, **characterized in that** the perforation line or perforation lines runs or run in the direction towards said further surface region (4).

5. The packaging as claimed in claim 4, **characterized in that** the perforation line or perforation lines or at least one of the perforation lines (6, 6') does not or do not extend as far as said further surface region (4).

6. The packaging as claimed in claim 4, **characterized in that** the perforation line or perforation lines or at least one of the perforation lines (6, 6') extends as far as said further surface region (4) or extends into this.

7. The packaging as claimed in any one of the preceding claims, **characterized in that** the carrier layer (8) is connected over the entire area to the covering layer (7), with the exception of said surface regions (3, 3'), and that between each of the two surface regions (3, 3') which form the spaces, enclosed on all sides, for receiving said films and the respectively adjacent outer margin of the packaging, there is a region within which the carrier layer (8) is connected to the covering layer (7) and which has a width of 3 to 7 mm, preferably 4-5 mm.

8. The packaging as claimed in any one of the preceding claims, **characterized in that** the two opposite surface regions (3, 3') which form the spaces for receiving said films have the same size and the same geometric shape, and are arranged symmetrically with respect to the longitudinal direction of the packaging, and **in that** the width of the web (5) located between the two surface regions (3, 3') corresponds to double the width of the margin which runs between an end-face outer margin of the packaging and the contiguous surface region which is intended for receiving said film.

9. The packaging as claimed in any one of the preceding claims, **characterized in that** said releasable connections are formed by sealing seams or sealing surfaces.

10. The packaging as claimed in any one of the preceding claims, **characterized in that** it has at least one folding or bending line (b) which runs in the longitudinal direction of the packaging and which touches or intersects said perforation (6).

11. Use of a packaging as claimed in any one of the preceding claims for the packaging of active substance-containing films, preferably for the child-proof packaging of active substance-containing films

12. A method for the packaging of active substance-containing films, the method having the following steps:
- providing a carrier layer (8);
- positioning of two active substance-containing films in a paired arrangement on the carrier layer (8);
- covering of the carrier layer (8) and of the active aubatance-containing films (1, 1') located on it with a covering layer (7);
- connection of the carrier layer (8) and covering layer (7) by means of a releasable connection, specifically in such a way that the two surface regions (3, 3') which enclose the active substance-containing films (1, 1') are surrounded completely by a marginal region in which the carrier layer (8) is connected to the covering layer (7), with the result that two spaces, which are enclosed on all sides and contain the two films, and a web (5) lying between these spaces, are formed, and in such a way that the connection of the carrier layer (8) and covering layer (7) takes place, furthermore, in such a way that the packaging has, in the region of the web (5) present between said two surface regions (3, 3'), a further surface region (4) in which the carrier layer (8) is not connected to the covering layer (7);
- generating at least one perforation line (6) within the web, **characterised in that** the perforation line (6) does not extend as far as the outer margin of the packaging, and that the sum of the surface regions in which the covering layer (7) is connected to the carrier layer (8) amounts to no more than 25%.

13. Method as claimed in claim 12, **characterised in that** the two opposite surface regions (3, 3') which form the spaces for receiving said films have the same size and the same geometric shape and are arranged symmetrically with respect to the longitudinal direction of the packaging to be produced, and **in that** the web (5) located between the two surface regions (3, 3') is designed in such a way that its width corresponds to double the width of the margin which runs between an end-face outer margin of the packaging and the contiguous surface region which is intended to receive said film.

14. The method as claimed in claim 12 or 13, **characterized in that** the connection between the carrier layer (8) and covering layer (7) is made by means of sealing, in particular using a peelable sealing lacquer.

15. The method as claimed in any one of claims 12 to 14, **characterized in that** a packaging having the features described in claims 1 to 10 is produced.

## Revendications

1. Emballage (10) pour films contenant une substance active (1, 1'), qui comprend une couche de support (8) et une couche de couverture (7) reliée à celle-ci de manière détachable,
- l'emballage comprenant, en disposition par paire, deux zones planes en vis-à-vis (3, 3') séparées l'une de l'autre par une partie intermédiaire (5), à l'intérieur desquelles la couche de couverture (7) n'est pas reliée à la couche de support (8), ce qui permet de former deux espaces séparés l'un de l'autre fermés sur tous les côtés pour la réception d'une paire desdits films (1, 1') ;
- une zone plane supplémentaire (4) étant présente à l'intérieur de ladite partie intermédiaire (5), zone dans laquelle la couche de support (8) n'est pas relié à la couche de couverture (7), ce qui permet de former un espace creux entouré sur tous les côtés ;
- au moins une ligne de perforation (6) étant présente à l'intérieur de la partie intermédiaire (5), **caractérisé en ce que** la ligne de perforation (6) n'atteint pas le bord extérieur de l'emballage et la somme des zones planes dans lesquelles la couche de couverture (7) est reliée à la couche de support (8) ne dépasse pas 25%.

2. Emballage selon la revendication 1, **caractérisé en ce que** la/les ligne/s de perforation ou au moins une des lignes de perforation (6, 6') est/sont placée/s non seulement dans la couche de support (8), mais également dans la couche de couverture (7).

3. Emballage selon la revendication 1 ou 2, **caractérisé en ce que** la/les ligne/s de perforation ou au moins une des lignes de perforation (6, 6') est/sont située/s dans la zone qui se trouve entre le bord extérieur de l'emballage et ladite zone plane supplémentaire (4).

4. Emballage selon l'une des revendications 1 à 3, **caractérisé en ce que** la/les ligne/s de perforation s'étend/ent en direction de ladite zone plane supplémentaire (4).

5. Emballage selon la revendication 4, **caractérisé en ce que** la/les ligne/s de perforation ou au moins une des lignes de perforation (6, 6') n'atteint/atteignent pas ladite zone plane supplémentaire (4).

6. Emballage selon la revendication 4, **caractérisée en ce que** la/les ligne/s de perforation ou au moins une des lignes de perforation (6, 6') atteint/atteignent ladite zone plane supplémentaire (4) ou pénètre/nt dans celle-ci.

7. Emballage selon l'une des revendications précédentes, **caractérisé en ce que** la couche de support (8) est reliée sur toute la surface à la couche de couverture (7), à l'exception des dites zones planes (3, 3'), et **en ce qu'**il existe, entre chacune des deux zones planes (3, 3') qui forment les espaces fermés sur tous les côtés destinés à recevoir lesdits films et le bord extérieur de l'emballage contigu à chacune d'elles, une zone à l'intérieur de laquelle la couche de support (8) est reliée à la couche de couverture (7) et qui mesure entre 3 et 7 mm, de préférence entre 4 et 5 mm, de largeur.

8. Emballage selon l'une des revendications précédentes, **caractérisé en ce que** les deux zones planes en vis-à-vis (3, 3') qui forment les espaces destinés à recevoir lesdits films ont la même grandeur et la même forme géométrique et sont disposées de manière symétrique par rapport à la direction longitudinale de l'emballage, et **en ce que** la largeur de la partie intermédiaire (5) située entre les deux zones planes (3, 3') correspond au double de la largeur du bord qui s'étend entre un bord extérieur du côté frontal de l'emballage et la zone plane contiguë destinée à recevoir ledit film.

9. Emballage selon l'une des revendications précédentes, **caractérisé en ce que** lesdites liaisons détachables sont formées par des joints de scellement ou des surfaces de scellement.

10. Emballage selon l'une des revendications précédentes, **caractérisé en ce qu'**il comprend au moins une ligne de pliage ou de pliure (b) qui s'étend dans la direction longitudinale de l'emballage et entre en contact avec ladite perforation (6) ou la coupe.

11. Utilisation d'un emballage selon l'une des revendications précédentes pour l'emballage de films contenant une substance active, de préférence pour l'emballage à l'épreuve des enfants de films contenant une substance active.

12. Procédé d'emballage de films contenant une substance active, le procédé comprenant les étapes suivantes :
- préparation d'une couche de support (8) ;
- positionnement de deux films contenant une substance active, en une disposition par paire, sur la couche de support (8) ;
- recouvrement de la couche de support (8) et des films contenant une substance active (1, 1') se trouvant dessus avec une couche de couverture (7) ;
- jonction de la couche de support (8) avec la couche de couverture (7) au moyen d'une liaison détachable, notamment de manière à ce que les deux zones planes (3, 3') qui enferment les films contenant une substance active (1, 1') soient totalement entourées par une zone de bord dans laquelle la couche de support (8) est reliée à la couche de couverture (7), ce qui permet de former deux espaces fermés sur tous les côtés qui contiennent les deux films, ainsi qu'une partie intermédiaire (5) située entre ces espaces, et en ce que la jonction de la couche de support (8) avec la couche de couverture (7) est réalisée, en outre, de manière à ce que l'emballage comporte, dans la zone de la partie intermédiaire (5) présente entre les deux dites zones planes (3, 3'), une zone plane supplémentaire (4) dans laquelle la couche de support (8) n'est pas reliée à la couche de couverture (7) ;
- production d'au moins une ligne de perforation (6) à l'intérieur de la partie intermédiaire, **caractérisé en ce que** la ligne de perforation (6) n'atteint pas le bord extérieur de l'emballage et la somme des zones planes dans lesquelles la couche de couverture (7) est reliée à la couche de support (8) ne dépasse pas 25%.

13. Procédé selon la revendication 12, **caractérisé en ce que** les deux zones planes en vis-à-vis (3, 3') qui forment les espaces destinés à recevoir lesdits films ont la même grandeur et la même forme géométrique et sont disposées de manière symétrique par rapport à la direction longitudinale de l'emballage à fabriquer, et **en ce que** la partie intermédiaire (5) située entre les deux zones planes (3, 3') est exécutée de manière à ce que sa largeur corresponde au double de la largeur du bord qui s'étend entre un bord extérieur du côté frontal de l'emballage et la zone plane contiguë destinée à recevoir ledit film.

14. Procédé selon la revendication 12 ou 13, **caractérisé en ce que** la liaison entre la couche de support (8) et la couche de couverture (7) est réalisée par scellement, notamment en utilisant un vernis de scellement pelable.

15. Procédé selon l'une des revendications 12 à 14, **caractérisé en ce qu'**un emballage possédant les caractéristiques décrites dans les revendications 1 à 10 est fabriqué.
